# EUROPEAN PATENT SPECIFICATION

(11) **EP 1 561 113 B1**
(45) Date of publication and mention of the grant of the patent: **17.08.2011**
(21) Application number: 03750379.4
(22) Date of filing: 26.09.2003
(51) Int. Cl.: G01N 33/574, G01N 33/573, G01N 33/48

(54) **A METHOD FOR DETECTING AND/OR SCREENING COLORECTAL CANCER IN AN INDIVIDUAL**
VERFAHREN ZUM NACHWEIS UND/ODER SCREENING ZUR ÜBERWACHUNG EINES KOLOREKTALKREBS IN EINEM INDIVIDUUM
METHODE DE DETECTION ET/OU DEPISTAGE DE SURVEILLANCE D'UN CANCER COLORECTAL CHEZ UN INDIVIDU

(30) Priority: 26.09.2002 DK 200201430
(43) Date of publication of application: 10.08.2005
(73) Proprietor: Hvidovre Hospital, 2650 Hvidovre (DK); Rigshospitalet, 2100 Copenhagen Ø (DK)
(72) Inventor: HESSEL, Lasse, L., DK-5700 Svendborg (DK); MALLING, Jesper, DK-5771 Stenstrup (DK); BRÜNNER, Nils, DK-2900 Hellerup (DK); HOLTEN-ANDERSEN, Mads, DK-2720 Vanlose (DK); NIELSEN, Hans, Jorgen, DK-2800 Lyngby (DK)
(74) Representative: Plougmann & Vingtoft A/S
(86) International application number: PCT/DK2003/000634
(87) International publication number: WO 2004/029627

(56) References cited:
- WO-A-00/62070
- WO-A-03/087830
- GARRETT C. DURKAN ET AL: "Prognostic Significance of Matrix Metalloproteinase-1 and Tissue Inhibitor of Metalloproteinase-ai in Voided Urine Samples from Patients with Transitional Cell Carcinoma of the Bladder" CLINICAL CANCER RESEARCH, vol. 7, November 2001 (2001-11), pages 3450-3456, XP002268767
- PATENT ABSTRACTS OF JAPAN vol. 1996, no. 09, 30 September 1996 (1996-09-30) & JP 08 136548 A (MORINAGA &CO LTD), 31 May 1996 (1996-05-31)

## Description

The present invention relates to a method for early detection and/or screening of a cancer in an individual.

A significant factor affecting the long term survival of cancer patients is the stage at which the cancer is detected. Early detection may facilitate rapid and complete removal of any malignancy before metastasis occurs as documented by increased cure rate and long-term survival of such patients.

Furthermore, if a cancer is detected, an early detection of the spread of cancer, the recurrence of cancer after treatment as well as the response to treatment, is crucial to the survival of cancer patients.

The best method of early detection is routine screening of those considered to be at risk. However, existing screening methods have met with variable success. It may be that they are too expensive and/or unreliable to be widely used. In any case, for many cancers, there is no practical detection method available. This problem, in combination with the fact that certain cancers, e.g. gastric and colorectal cancers, exhibit symptoms consistent with much more common non-malignant pathologies, make them difficult to detect at an early stage.

Colorectal cancer (CRC) is currently the fourth most common cancer in the US and ranks second as a cause of cancer-related deaths. Evidence exists that reductions in colorectal cancer morbidity and mortality can be achieved through detection and treatment of early-stage colorectal cancers and the identification and removal of adenomatous polyps, the precursors of colorectal cancer.

Currently, the only way of diagnosing colorectal cancer is to carry out a colonoscopy, which is impractical as a screening technique for general applicability. Furthermore, since the disease is still primarily managed by surgical resection, there has been great interest in screening.

A known screening test for colorectal cancer that is often recommended on a routine basis by many health-care organizations is the fecal occult blood test (FOBT), which uses various methods to detect blood in the stool. However, although the test has a high specificity, this method has an intermediate sensitivity often leading to either false-positive or false-negative results, and may therefore cause unwarranted anxiety about cancer and unnecessary further tests or may miss the disease in its early stages.

The American Society of Clinical Oncology (ASCO) has therefore recommended against the use of fecal occult blood tests as a means for regular postoperative monitoring of colorectal cancer, (Benson et al, JCO, 2000). ASCO has also emphasised that new surveillance methods for the detection of colorectal cancer recurrences are needed (Benson et al, JCO, 2000).

Because metastatic disease is the main cause of cancer patient morbidity and mortality, molecules involved in the regulation of tumour invasion and metastasis are attractive as potential screening/detection/monitoring targets. It is well established that proteolytic enzymes produced by cancer cells or by cells in the tumour stroma are involved in extracellular tissue degradation, leading to cancer cell invasion and metastasis. A number of enzymes have been associated with this process, the most thoroughly investigated being the metalloproteinases, such as the collagenases and stromelysins, and the serine proteases such as plasmin. Recently, data has been published indicating that these molecules, free or bound in complexes, are released from tumour tissue and find their way into the circulation.

A positive correlation between tumour cell aggressiveness and matrix metalloproteinases (MMPs) expression has been well documented. MMPs are a group of zinc-binding endopeptidases involved in connective tissue matrix remodelling and degradation of the extracellular matrix (ECM), which are essential steps in tumour invasion, angiogenesis, and metastasis (reviewed by Aznavoorian et al, 1993).

However, matrix metalloproteinase enzyme activity is highly regulated. Regulation occurs at multiple levels, including modulation of MMP gene expression, extracellular activation of the pro-enzyme, inhibition of activation and enzymes activity by association with tissue inhibitors of metalloproteinase (TIMPS) (Matrisian, 1990), (Mauviel, 1993), (Birkedal-Hansen, 1993).

TIMPs forms stoichiometric complexes with both latent and active MMP's (Welgus *et al*., 1985; Kleiner *et al*., 1993), thereby inhibiting the catalytic activity of these enzymes (Stetler-Stevenson *et al*., 1996; Goldberg *et al.,* 1992; Birkedal-Hansen *et al*., 1993).

TIMPs represent a family of ubiquitous proteins. There have, at present, been identified four members of the TIMP family. It has been found that TIMP-1 and TIMP-2 are capable of inhibiting tumour growth, invasion, and metastasis (Liotta, *et al*., 1991 and Khokha *et al*., 1989). However, recent clinical studies have stated TIMP-1 and TIMP-2 to be rather tumour-promoting molecules, as they were found to be significantly overexpressed in patients with poor prognosis. (McCarthy et al, 1999; Remacle et al, 2000).

WO 03/087830 discloses a method for determining whether an individual is likely to have minimal residual disease, recurrent cancer or a combination of minimal residual disease and recurrent cancer after being treated for the primary cancer. The first parameter may be the total concentration of TIMP-1 and the body fluid sample may be saliva.

Garrett et al. discloses that increased levels of urinary TIMP-1 are associated with bladder cancer and disease progression in bladder cancer.

Patent abstracts of Japan vol. 1996, no 09, 30 September 1996 & JP 08 136548 discloses a method for diagnosing urinary organ cancer by determining TIMP-1 in urine.

Studies have shown that TIMP-1 in plasma is a highly sensitive and specific screening marker for the identification of patients with a high risk of having colorectal cancer (Holten-Andersen *et al*., 1999 and Holten-Andersen *et al*. 2002).

A method using this information is disclosed in WO00/62070 . . In said reference it has been found that individuals can be screened for colorectal cancer by measuring the TIMP-1 concentration in plasma samples.

However, the method has the drawback that a blood sample must be drawn for each individual. This can be extremely expensive and troublesome when detecting and monitoring cancers in large populations and in some cases even impossible e.g. in the developing countries.

Furthermore, trained staff must draw blood samples, and particular care must be taken with the samples, as there is a high prevalence of hepatitis B and the human immunodeficiency virus (HIV) infections in some populations, e.g. in the developing countries.

Based on these problems other fluids mentioned in the exhausted list of WO/0062070 such as urine and serum has been evaluated as an alternative to plasma, as these are the traditional biological samples for the qualitative and quantitative measurement of substances including proteins.

However, serum studies have shown that there is no significant difference between the total TIMP-1 concentrations in serum samples from individuals having colorectal cancer and healthy blood donors, most properly because the platelets will leak TIMP-1 during coagulation *in vitro* (Cooper et al, 1985; Jung et al, 1996). Furthermore, in other studies, TIMP-1 has by itself been proven inadequate as a serum marker for screening for pancreatic cancer, (Zhou et al, 1998).

The concentration of TIMP-1 in urine and plasma from the same colorectal cancer patients has also been evaluated, but without finding any noticeable correlation, since no TIMP-1 elevation was found in the urine (Brünner, unpublished data).

It is known for the person skilled in the art, that all organic compounds of plasma, such as hormones, immunoglobulines, enzymes, proteins, DNA and viruses may be detected in saliva in trace amounts (Vining and McGinley, 1985). However, it is a well established knowledge that the concentration found in saliva is lower than the concentration in urine which again is lower than the concentration found in plasma.

The lower concentrations in saliva are caused by a number of factors. As an example can be mentioned that saliva contains enzymes for digestion and that the total protein concentration in saliva is negligible since it is less than 1% of that in plasma (Breimer and Danhof, 1980). Furthermore it is likely that a major source of these trace amounts originates from the gingival crevicular fluid (from the tooth/gum margin) (Cimasoni, 1974).

A person skilled in the art would therefore expect that the natural enzymes, such as proteases in the saliva, would digest these negligible protein concentrations and reduce any concentration found in e.g. saliva to a negligible concentration if detectable at all.

One of a number of examples of a protein having a lower concentration in urine than in serum/plasma and again an even lower concentration in saliva, is prostate-specific antigen (PSA), which is the most commonly used serum marker for screening for prostate cancer, (Balk et al, 2003). PSA is present in high concentrations in serum and plasma, in minor concentrations in urine, and in one study no detectable PSA levels have been found in saliva at all, (Lovgren et al, 1999). Furthermore, another study has shown that determination of free and total PSA in saliva to improve and simplify the differentiation between prostate cancer and benign prostatic hyperplasia is not suitable for use as an alternative measurement of serum (Turan et al, 2000).

Accordingly, since no elevated levels of TIMP-1 could be found in urine of either healthy blood donors or individuals suffering from colorectal cancer, the person skilled in the art would based on the common general knowledge that the concentration of a protein in saliva always would be lower than in urine and plasma have no hint to examine saliva as a possible body fluid for detection of TIMP-1.

It should in this respect be noted, that TIMP-1 can be found in sputum from patients with asthma and chronic bronchitis (Vigonola et al, 1998), but TIMP-1 is in such cases coming from blood from small lesions in e.g. the lung tissue.

Thus, as the current available screening methods for colorectal cancer are based on blood or stool samples, there is a long felt need for a method for detecting, screening and monitoring cancers, which is applicable for large populations without the need to use blood or stool samples.

It is therefore one aspect of the present invention to provide a screening method, which is suitable to facilitate the early diagnosis of colorectal cancer without the need to use blood or stool samples, in an individual that has no prior history of cancer. This is achieved according to the invention as it has surprisingly been found by the inventors that the TIMP-1 protein can be detected in saliva in unexpected high concentrations with respect to the TIMP-1 concentration in plasma.

The inventors hereby overcome the common prejudice that a protein cannot be detected in saliva if it has not been detected in urine. As previously stated, earlier studies have shown, that noticeable concentrations of TIMP-1 protein could not be detected in urine and there has therefore been no indication in the prior art for the skilled person to look for TIMP-1 in saliva.

Furthermore, as a non-invasive technique, the collection and analysis of the excreta would appear to be particularly attractive for a high-risk patient population where the routine collecting of blood is often made difficult because of bruised or thrombosed veins.

In addition, this would be beneficial to both the patient (blood sampling would be reduced) and to those who handle patient samples (clinic and laboratory staff) e.g. because saliva can inhibit HIV infectivity (Wolff and Hay, 1991).

It is therefore possible according to the present invention to provide an easy and inexpensive method for early detection and screening of colorectal cancer in an individual or large populations using a non-invasive technique for collection of samples.

In order not to obtain any false-positive results it is important to determine a discriminating value, which divides the tested individuals in a group having either a high or low likelihood of having cancer.

It has been found that in one embodiment of the invention the total concentration of TIMP-1 is measured, that is, the sum of the TIMP-1 in free form and the TIMP-1 in complex forms. The person skilled in the art will understand that other expressions than the exact concentration can represent the concentration, such as, e.g., the concentration multiplied by a factor, etc., and that such other representations can be used equally well for the purpose of the present invention provided the corresponding adjustments are made.

The discriminating value is established by measuring the total concentration of TIMP-1 in both a healthy control population and a population with known cancer and thereby determining the discriminating value. The discriminating value identifies the cancer population with either a predetermined specificity or a predetermined sensitivity or both, and is based on an analysis of the relation between the concentration values and the known clinical data of the healthy control population and the cancer patient population.

The discriminating value determined in this manner is valid for the same experimental set-up in future individual tests.

Specificity is defined as the proportion of non-diseased individuals having a parameter representing the concentration of a marker, such as TIMP -1 in excreta samples lower than a predefined level.

Sensitivity is defined as the proportion of patients having colorectal cancer having a parameter representing the concentration of a marker, e.g. TIMP-1 in excreta samples higher than a predefined level.

Studies have shown that an increase in detection and monitoring values can be obtained when combining the concentration of total TIMP-1 and the concentration of free TIMP-1, thereby providing a highly selective and sensitive method for detecting, screening and monitoring an individual for cancer.

Such cancers could e.g. be breast, prostate, colorectal, cervical, ovarian, lung, pancreatic, renal, vulvar, hepatocellulaar carcinomas, minimal residual disease and recurrent cancer

In order to improve the sensitivity of the method according to the invention, an additional marker can optionally be employed. Said marker is preferably Carcino Embryonic Antigen (CEA), which is a protein, which is normally found only during foetal development, but may reappear in adults who develop certain types of cancer, primarily cancer of the gastrointestinal system. However, other markers for colorectal cancer known for the person skilled in the art, such as soluble u-PAR, could equally well be employed.

Preferably logistic regression analysis can be used when combining either the concentration of total TIMP-1 and the concentration of free TIMP-1 combination or the combination of concentrations of the cancer marker (CEA) and TIMP-1.

Data has shown that by adding CEA as the additional marker, an improvement in the sensitivity of total TIMP-1 in plasma can be obtained and maintained with a very high specificity. Thus, the combination of concentrations of CEA and TIMP-1 could be useful in a method for identifying patients with a high risk of having cancer.

The method according to the invention may be used both for an individual and for an entire population, but more appropriately to a population already identified as having an increased risk of developing cancer, e.g. individuals with a genetic disposition, individuals who have been exposed to carcinogenic substances, or individuals with cancer-predisposing non-malignant diseases.

As an example this could e.g. be individuals with a prior polyp, individuals with Crohn's disease or ulcerative colitis, individuals with one or more family members with colorectal cancer .

When an individual has been identified as having high TIMP-1 levels in his or her excreta, the individual should be referred for further examination. If a cancer is found, the patient could be offered surgery, radiation or adjuvant anti-neoplastic therapy aiming at curing the patient of cancer.

The application further relates to a dipstick for measurement of the concentration of TIMP-1 either alone or in combination with CEA, in excreta.

Such a dipstick is extremely easy and quick to use and each individual can perform the test without the need of a professional staff or the use of specialist equipment.

Individuals in certain high-risk groups, such as individuals having e.g. colitis ulcerosa or individuals with an earlier detected cancer who wants to monitor a possible recurrence of the cancer, then have the possibility of screening/monitoring themselves at any desired frequency.

Furthermore, the dipstick can be a valuable tool since each individual has the opportunity to monitor the response to treatment and the spread of cancer without the need for specialist equipment.

The dipstick is preferably a two-sited direct label assay in a lateral flow device format.

In a first embodiment the dipstick can be made of a capillary material, and comprises a first colour indication zone comprising antibodies specific for TIMP-1 and at least one reagent which can give an optically visible colour change in the zone dependent on the concentration of TIMP-1 in the tested excreta.

The dipstick further comprises a second colour indication zone, able to react with at least one substance normally present in the excreta, and thereby providing an optically visible colour change in the zone for controlling that the stick is used properly.

In another embodiment of the dipstick , the dipstick further comprises a third colour indication zone, comprising antibodies specific for CEA and at least one reagent which can give an optically visible colour change in the zone dependent on the concentration of CEA in the excreta, ensuring a more reliable screening method.

Preferably there is, in combinations with the dipstick, provided a colour reference that indicates the discrimination value for the specific excreta.

When the user examines the likelihood of having a cancer, the dipstick is first brought into contact with the excreta being tested, e.g. the user can place the dipstick in the mouth thereby bringing it into contact with the patient's saliva.

After a certain reaction period determined by the specific reagents on the dipstick, the user can compare the stick with the colour reference scale, dividing the patient into a group with either a high or a low likelihood of having cancer.

The dipstick can be based on any known conventional detection principle based on for instance RIA or enzymatic assays.

A person skilled in the art would recognise that the dipstick is only one possibility of detecting TIMP-1 in excreta, such as saliva. Other options are e.g. an Activity Assay (such as zymography), immunologic assays or a Colour Reaction kit.

The invention will be explained in greater detail below, by way of examples only with reference to the drawing, in which
Fig. 1 shows the recovery of TIMP-1 signal, measured following addition of increasing concentrations of purified TIMP-1 to a panel of 0.5% and 1% saliva pools in sample dilution buffer,
Fig. 2 shows total TIMP-1 concentration from 39 dilutions of a saliva pool from collection (I) assayed via ELISA, and
Fig. 3 shows the TIMP-1 concentration in each saliva sample from collection (I) measurement via ELISA.

### EXAMPLES:

It should initially be mentioned that even though the concentration of TIMP-1 in both plasma and saliva in the following examples are presented as ng TIMP-1 per volume plasma or saliva, it could equally well be presented as e.g. unit or gram TIMP-1 per unit or gram of a known reference molecule.

### Donors/Patients

In collection (I), saliva was obtained from four apparently healthy volunteer blood donors.

In collection (II) saliva was obtained form four apparently healthy volunteer blood donors. A simultaneous plasma sample was obtained from each individual at the time of saliva collection.

In collection (III) saliva was obtained form three patients with known colon or rectal cancer. A simultaneous plasma sample was obtained from each individual at the time of saliva collection.

Informed consent was obtained from all donors/patients, and permission was obtained from the local Ethical Committees.

### Saliva collection

In collection (I) saliva was collected from healthy blood donors who were instructed to rinse the mouth with tap water just before the collection. The collection duration was 5 minutes and the donor delivered in this period approximately five saliva samples in a 50 ml Nunc test tube. The saliva is centrifuged 5 minutes at 4000 rpm and the supernatant is then centrifuged at 15000 rpm for 5 minutes.

In collection (II) and (III), saliva was collected by introducing a cotton tampon into the mouth for 3 minutes. The tampon was then centrifuged 5 minutes at 3000 rpm to allow the saliva to be extracted. Subsequently the saliva was centrifuged for 15.000 rpm for 5 minutes.

The resulting supernatant is in all cases frozen at -20°C, until it is used.

### Blood collection and plasma separation

Peripheral blood was drawn with minimal stasis (if necessary a maximum of 2 min stasis with a tourniquet at maximum +2 kPa was acceptable) into pre-chilled citrate, EDTA, or heparin collection tubes (Becton-Dickinson, Mountain View, CA), mixed 5 times by inversion, and immediately chilled on ice. As soon as possible (no later than 1.5 h after collection) the plasma and blood cells were separated by centrifugation at 4°C at 1,200 x g for 30 min, and stored frozen at -80°C prior to assay. Plasma pools were made with freshly collected samples from at least ten donors, aliquoted and stored frozen at -80°C. For analysis, the samples were quickly thawed in a 37°C water bath and placed on ice until needed.

### Example 1: Measurement of total TIMP-1 concentration in saliva

### Preparation of an ELISA to quantitate total TIMP-1 concentrations in human plasma.

A sensitive and specific sandwich ELISA was prepared, using TIMP-1 antibodies developed at the Strangeways Laboratories (Hembry *et al*, 1985). A sheep polyclonal anti-TIMP-1 antiserum (Hembry *et al*, 1985; Murphy *et al,* 1991) was used for antigen capture, and a murine monoclonal anti-TIMP-1 IgG1 (MAC-15) (Cooksley *et al*, 1990) for antigen detection.

A rabbit anti-mouse immunoglobulin/alkaline phosphatase conjugate (Catalog number D0314, Dako, Glostrup, Denmark) was the secondary detection reagent. The latter conjugate was supplied preabsorbed against human IgG, thus eliminating cross-reactivity with IgG in the samples.

As the monoclonal detection antibody MAC-15 recognises both free TIMP-1 and TIMP-1 in complex with MMP's (Cooksley *et al,* 1990), the total TIMP-1 content captured by the sheep polyclonal anti-TIMP-1 antiserum was quantitated by the ELISA.

96-well microtiter plates (Maxisorp, Nunc, Roskilde, Denmark) were coated for 1 h at 37°C with 100 µL/well of polyclonal sheep anti-TIMP-1 (4 mg/L) in 0.1 mol/L carbonate buffer, pH 9.5. The wells were then rinsed twice with 200 µL/well of SuperBlockJ solution (Pierce Chemicals, Rockford, IL) diluted 1:1 with phosphate-buffered saline (PBS). The microtiter plates were stored for up to 14 days at -20°C. On the day of analysis, the plates were thawed at room temperature and washed 5 times in PBS containing 1 g/L Tween.

A series of purified, recombinant human TIMP-1 standards were used to calibrate each plate. Standards were prepared by serially diluting a stock solution of purified TIMP-1. Standard concentrations were 5, 3, 2, 1, 0.5, 0.25, 0.1, -0 ng/mL. Included on each plate was a blank containing only sample dilution buffer, and 2 controls made from a 1:100 dilution of a citrate plasma pool. One control was added as the first sample on the plate and the second control was added as the last.

All samples were diluted 1:100 or 1:51 in sample buffer consisting of 50 mol/L phosphate, pH 7.2, 0.1 mol/L NaCl, 10 g/L bovine serum albumin (Fraction V, Boehringer-Mannheim, Penzberg, Germany), and 1 g/L Tween 20.

A total of 100 µL/well of each standard, blank, control, and patient sample was incubated on the plate for 1 h at 30°C. All standards, blanks, controls, and samples were run in triplicate on each plate for every assay.

After primary incubation, the wells were washed 5 times, then treated for 1 h at 30°C with 100 µL/well of purified MAC-15 monoclonal antibody (0.5 mg/L) in sample dilution buffer. After another 5 washes the wells were incubated for 1 h at 30°C with 100 µL/well of rabbit anti-mouse immunoglobulins(Ig)/alkaline phosphatase conjugate diluted 1:2000 in sample dilution buffer.

Following 5 washes with washing solution and 3 washes with distilled water, 100 µL of freshly made p-nitrophenyl phosphate (Sigma, St. Louis, MO) substrate solution (1.7 g/L in 0.1 mol/L Tris.HCl, pH 9.5, 0.1 mol/L NaCl, 5 mmol/L MgCl₂) were added to each well.

The plate was placed in a Ceres 900J plate reader (Bio-Tek Instruments, Winooski, VT) at 23°C with the yellow colour development automatically monitored. Readings were taken at 405 nm against an air blank every 10 min. for one hour. KinetiCalc II software was used to analyse the data by calculating the rate of colour formation for each well (linear regression analysis), generating a 4-parameter fitted standard curve, and calculating the TIMP-1 concentration of each sample.

### Recovery experiments

The recovery of the TIMP-1 signal was measured following addition of increasing concentrations of purified TIMP-1 to a panel of 0.5% and 1% saliva pools in sample dilution buffer, followed by subsequent measurement of the signal.

The purified TIMP-1 was added to dilution series of the saliva pools to give concentrations in the range of 0 to 5 ng/mL.

The recovery was in each case calculated from the slope of the line representing the TIMP-1 signal as a function of concentration, where 100% recovery was defined as the slope obtained when TIMP-1 was diluted in sample dilution buffer.

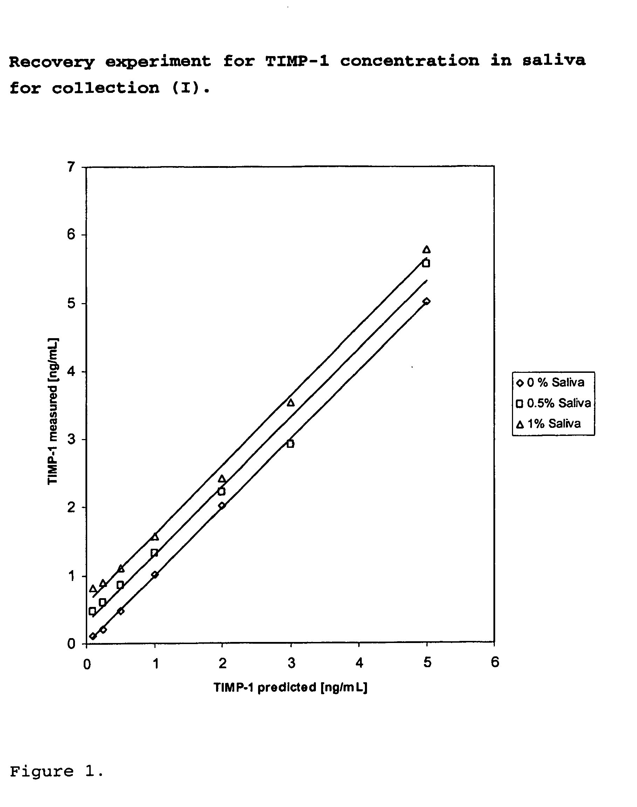

Recovery was 100,2% in 0,5% saliva and 100,9% in 1% saliva (Figure 1). Thus the recovery of TIMP-1 signal from an internal standard was excellent for all preparations of saliva, and the saliva samples all gave good linearity of the signal as a function of dilution.

### Measurement of Total TIMP-1 concentration in collection (I)

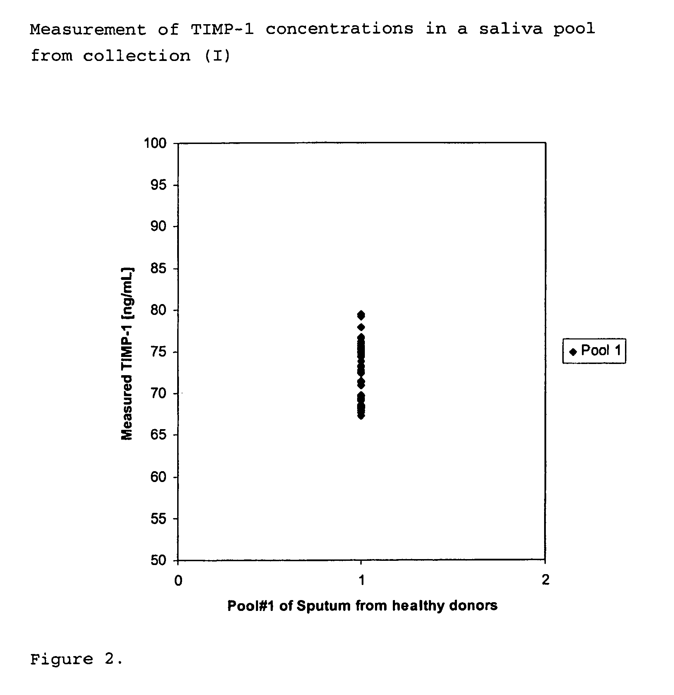

Saliva samples from collection (I) were pooled and 39 dilutions were assayed for total TIMP-1 concentration via ELISA (Figure 2). The mean TIMP-1 concentration in saliva was 72.81 ng/mL ± 3.33 with a reference range from 67.29 to 79.49 ng/mL.

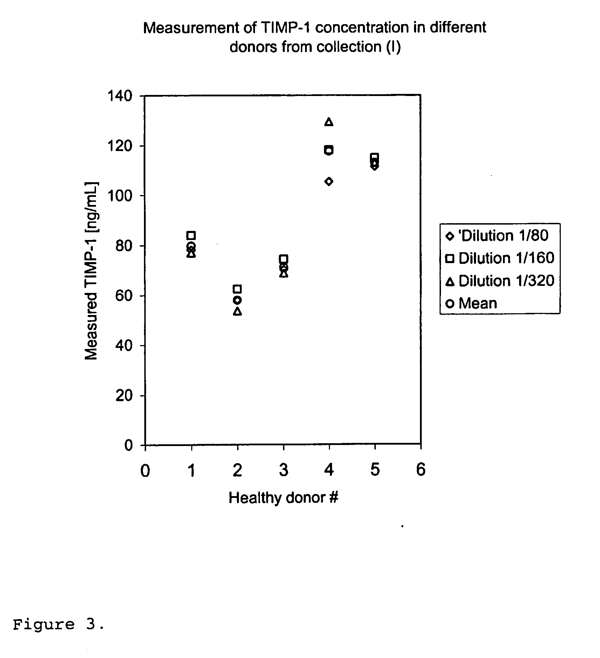

Each saliva sample from collection (I) as well as a pool of the samples was further assayed for total TIMP-1 concentration via ELISA. The mean TIMP-1 concentration in saliva was 79.66 ng/mL + 3.77 with a reference range from 76.96 to 83.96 ng/mL (Figure 3).

### Measurement of Total TIMP-1 concentration in collection (II)

In collection (II) both the saliva samples and the plasma samples from healthy blood donors were assayed for total TIMP-1 concentration via ELISA. The results are presented in table 1:

**Table 1: Total TIMP-1 concentration (ng/mL) in saliva and plasma in four healthy blood donors.**

| Healthy donor | Total TIMP-1 concentration in saliva (ng/mL) | Total TIMP-1 concentration in EDTA plasma (ng/mL) |
|---|---|---|
| 1 | 162 | 86,4 |
| 2 | 143,8 | 76,5 |
| 3 | 87,8 | 82,7 |
| 4 | 90,5 | 88 |

### Discussion

The assay described above enables accurate determination of total TIMP-1 in human saliva samples. The use of a rapid blocking agent and a dilution buffer with high buffering capacity also contributed to reproducible assays. Incorporating all these elements in the final assay fulfilled the requirements of sensitivity, specificity, stability, and good recovery of an internal standard.

The quantitative studies in saliva from healthy donors showed that saliva samples are suitable for TIMP-1 determination.

These studies showed that for collection (I) TIMP-1, levels in saliva (mean 72-79 ng/mL) correspond to TIMP-1 levels in plasma (mean 65-70 ng/mL) (see International Patent Application No. WO00/62070). Similar results were obtained from the healthy donors in collection (II).

### Example 2: Detection value of total TIMP-1 in patients with colorectal cancer.

Total TIMP-1 levels in both plasma and saliva from three colorectal cancer patients (collection (III)) were measured with the TIMP-1 assay described in Example 1. The TIMP-1 values were analyzed and compared using standard biostatistical parameters.

### Patients

Three patients undergoing elective surgery for pathologically confirmed colorectal cancer were included in the study. Blood and saliva samples were obtained preoperatively with informed consent from all patients in accordance with the Helsinki declaration, and permission was granted by the local Ethical Committee of Hvidovre Hospital, Denmark. All patients had pathologically verified adenocarcinoma of the colon or rectum.

### Measurement of Total TIMP-1 concentration in collection (III)

In collection (III) both the saliva samples and the plasma samples from colorectal patients were assayed for total TIMP-1 concentration via ELISA. The results are presented in table 2:

**Table 2: Total TIMP-1 concentration in saliva and plasma in ng/mL in three colorectal patients.**

| Colorectal patient | Total TIMP-1 concentration in saliva (ng/mL) | Total TIMP-1 concentration in EDTA plasma (ng/mL) |
|---|---|---|
| 5 | 328 | 395,7 |
| 6 | 409,9 | 127 |
| 7 | 358,9 | 172,3 |

### Discussion

This data proved that similar saliva/plasma TIMP-1 concentrations were found not only for healthy individuals but also in patients suffering from colorectal cancer.

Furthermore, all three colorectal cancer patients had elevated (above the 95 percentile of healthy blood donor plasma levels) saliva and plasma TIMP-1 levels. There is therefore a highly statistical difference in the total saliva TIMP-1 values between the colorectal cancer patients and the healthy blood donors.

It has previously been found, that a highly statistical difference in the total plasma TIMP-1 values between the colon and rectal cancer patients each compared with the healthy blood donors exists. Furthermore, it has been shown in the aforementioned examples that corresponding concentrations of total TIMP-1 is present in saliva and plasma. The person skilled in the art would based on these finding therefore expect that the same statistical difference between the colon and rectal cancer patients would be observed in total TIMP-1 from saliva.

On a similar basis the person skilled in the art could use the measured total TIMP-1 levels in saliva from healthy donors and the colorectal cancer patients, and generate Receiver Operating Characteristics (ROC) curves to evaluate the diagnostic value of total TIMP-1. As established in WO00/6.2070 , such curves have established high sensitivity and specificity of total TIMP-1 in plasma as a marker for colorectal cancer. Based on the above findings similar results are expected for total TIMP-1 in saliva.

This data suggest that total TIMP-1 measurements in saliva can be used as a screening procedure to aid in identifying patients with a high risk of having colorectal cancer.

In particular, total TIMP-1 in plasma has in WO00/62070 proven effective in identifying patients with early cancer (Duke's stage A+B) as well as identifying patients with more advanced disease. Based on the findings in the present invention similar results would be expected for total TIMP-1 concentrations in saliva. Similarly, as total TIMP-1 in plasma was more effective in identifying patients with early stage, right-sided colon cancer, the same would be expected for total TIMP-1 in saliva. Right-sided colon cancer cannot be visualized by flexible sigmoidoscopy, a standard colon cancer screening methodology. It has a more insidious onset than left-sided lesions do, and clinical symptoms develop only in the later stages of the disease. Early diagnosis of right sided colon cancer has the potential to reduce the mortality of this disease.

The optimal discriminating value for TIMP-1 in saliva to discriminate between malignant and normal tissues can be determined on the basis of such ROC curves as the point on the curve, which shows the optimal combination for specificity and sensitivity and where diagnostic accuracy is maximized. The discriminating value for total TIMP-1 in plasma based on 870 healthy blood donors has been determined (Brünner, unpublished data), and based on the above findings a similar result is expected for total TIMP-1 in saliva.

### Example 3: Measurement of the total concentration of TIMP-1:MMP-9 complexes and free TIMP-1 in saliva

As shown in Example 1 the total TIMP-1 concentration in saliva corresponds very accurately to the total TIMP-1 concentration in plasma. As it has been found that there exists a correlation between plasma and saliva, it would be obvious to the person skilled in the art to also examine the total concentration of TIMP-1:MMP-9 complexes and free TIMP-1 concentration in saliva. Appropriate methods and materials are described in similar examples in WO00/62070 . In view of the findings in Example 1, it would be expected that similar results would be obtained for the total concentration of TIMP-1:MMP-9 complexes and free TIMP-1, respectively.

### Example 4: Detection value of total TIMP-1 in combination with CEA in patients with colorectal cancer.

Examples in WO00/62070 have demonstrated that by adding an additional marker, an improvement in the diagnostic sensitivity of total TIMP-1 in plasma can be obtained, while maintaining a high specificity of 98%. Thus, based on the assumption that corresponding data can be obtained with total TIMP-1 in plasma and saliva, the combination of CEA and TIMP-1 in saliva could be useful as a screening procedure to identify patients with a high risk of having colorectal cancer.

### Example 5: Lack of detection value of total TIMP-1 in saliva in patients with primary (stage I and II) breast cancer.

Using the total TIMP-1 assay described in Example 1, total TIMP-1 levels in pre-operative plasma samples from 322 patients with primary breast cancer were compared with 108 plasma samples from healthy blood donors. Data shown in WO00/62070 , showed no statistical significant difference in total TIMP-1 plasma levels between the two groups (Mann-Whitney, p=0.87). Thus, this data supports the specificity of TIMP-1 measurements in the diagnosis of patients with colorectal cancer. Similar results would be expected if the total TIMP-1 level were measured in saliva samples from similar donors.

### Example 6: Detection value of saliva total TIMP-1 in patients with metastatic (stage IV) breast cancer.

Based on information from WO00/62070 where total TIMP-1 levels were measured in 19 breast cancer patients with stage IV disease all EDTA plasma samples using the assay described in Example 1. These levels were compared with total TIMP-1 plasma levels in 87 healthy female donors. A Wald-Wolfowitz test indicated a highly significant difference (p<0.0001) between patient total TIMP-1 levels and those of healthy donors. As it has been established that corresponding levels of total TIMP-1 are present in plasma and saliva these data show that saliva TIMP-1 measurements can be used to monitor breast cancer patients for recurrence of disease.

### Example 7: Detection value of free TIMP-1 in saliva in patients with colorectal cancer.

In WO00/62070 is was shown that free TIMP-1 in plasma alone is not likely to be useful as a screening marker to identify patients with a high risk of having colorectal cancer. As previously mentioned based on the results shown in the present application similar results are expected for free TIMP-1 in saliva.

### Example 8: Screening value of a dipstick for detection of TIMP-1 in saliva.

### Procedure

A colloidal gold/antibody conjugate was produced for practice of the methods of the present invention. Siliconized glassware (Sigma silicote) was utilized throughout the procedure wherein 200 ml of 0.01% gold chloride (HAuCl 4.3H₂O) (Fisher Scientific, G-54-1) was brought to a boil and 2 ml of 1% sodium citrate solution was added and the boiling is continued for 5 minutes until the color of the solution changes from pale yellow to purple to red. A solution of potassium carbonate (0.02M) was added to the suspension in order to adjust the pH to 7.6, followed by addition of the rabbit polyclonal anti-MMP-9 antibody (1 mg/ml) (Novus Biologicals Product) such that approximately 10 µg IgG was added per ml of gold suspension (0.01% gold).

After one minute of incubation at room temperature, 0.1 ml of a solution of 30% bovine serum albumin in water was added to 10 ml of the gold suspension. Aggregated material was removed by centrifugation at 3000 rpm in the SS34 rotor of a Sorval RC5C centrifuge for 10 minutes. The supernatant was subjected to an additional centrifugation step at 6000 rpm for one hour. The colloidal gold conjugate in the pellet was resuspended in 2% bovine serum albumin in PBS (0.05 M potassium phosphate buffer, pH 7.4, in 0.9% NaCl), the preferred conditions for liquid storage being at 4°C. Casein solution was added to give a concentration of 1% casein immediately before application to the membrane. The conjugate was then stored for prolonged periods in the dry state, with no loss of activity after 6 months storage at 37° C. in the dry state.

According to this example, sandwich-type immunoassay devices for the detection of TIMP-1 were constructed and used. Microporous nitrocellulose material with a thickness of approximately 0.1 mm and an average pore size of 5 µm was laminated with mylar and adhesive (Monokote, Top Flite Models, Inc., Chicago, Ill.). Strips measuring 1 cm by 3.5 cm were cut. Mouse monoclonal antibody MAC 19 (see WO/62070), 0.2 µl, was applied to the strips at the capture zone where it was immobilized and air dried. Non-specific binding sites on the chromatographic strip materials were then blocked by incubation for 10 minutes at room temperature with a 0.1% solution of LB gelatin in water (Inotech, Wohlen, Switzerland) and the strips were allowed to dry under a stream of air. One µl of gold particle labeled rabbit polyclonal anti-MMP-9 antibody in an anti-aggregation buffer produced as described was then applied to a first zone of each strip and dried.

### Detection of TIMP-1 concentration in saliva samples on a dipstick

Saliva samples from collection (II) and (III), healthy blood donors and colorectal cancer patients, respectively, were first applied to the second zones between the first and third zones.

The first end of the strips were then dipped into a chromatographic transport solvent comprising TBS and 1% TRITON X-100 (polyethylene glycol tert-octylphenyl ether). The liquid front was allowed to progress to the second ends of the devices over a period of approximately 2.5 minutes transporting the sample material and the gold-labeled goat anti-human IgG to the third zone.

Samples from the colorectal cancer patients (collection (III)) and the immobilization of the labeled first reagent resulted in the presence of a red spot at the third zone. Strips tested with samples from the healthy blood donors, collection (II) did not produce a signal at the third zone.

### Discussion

These test confirm that TIMP-1 can be measured in saliva by means of a dipstick, and that such a dipstick can be used in a screening procedure to aid in identifying patients with a high risk of having colorectal cancer. Furthermore, the dipstick can be used to monitor the response to treatment and the spread of cancer without the need for specialist equipment.

### CONCLUSION

The person skilled in the art would understand from the present invention that the similarities between total TIMP-1 levels in plasma and saliva is not limited to the Examples mentioned in the current application. In fact it is now surprisingly expected that for any TIMP-1 value measured in a plasma sample a corresponding value could be established for TIMP-1 in saliva.

This is not just true for the examples mentioned in the present application or in WO00/62070 but also for all other known results obtained for plasma.

The data presented in the present application supports the unique value of testing saliva when screening, monitoring and detecting early stage colorectal cancer or metastatic breast cancer using TIMP-1 as a cancer marker. The method for identification of patients having primary colorectal cancer is highly specific, and patients with non-malignant conditions, such as inflammatory bowl diseases, are not detected. Furthermore, saliva may also be suitable when monitoring the response to treatment and the progress of cancer.

The description has primarily described excreta from humans; however it is within the scope of the invention that the excreta is from an animal, e.g. a dog, cat or cow.

### REFERENCE LIST

**Agency for Health Care Policy and Research,** Rockville, MD; Colorectal Cancer Screening. Summary, Evidence Report: Number 1. AHCPR Publication No. 97-0302.
Aznavoorian S, Murphy AN, Stetler-Stevenson WG, Liotta LA, (1993); Molecular aspects of tumor cell invasion and metastasis. Cancer 15;71(4):1368-83, rewiev.
Balk, S.P., Ko, Y. and Bubley, G 2003, Biology of Prostate-Specific Antigen, Journal of Clinical Oncology, Vol 21, Issue 2 (January), 2003: 383-391
Benson, A.B., Desch, C.E., Flynn, P.J., Krause, C., Loprinzi, C.L., Minsky, B.D., Petrelli, N.J., Pfister, D.G., Smith, T.J., Somerfield, M.R., (2000) for the American Society of Clinical Oncology; 2000 Update of American Society of Clinical Oncology Colorectal Cancer Surveillance Guidelines; Journal of Clinical Oncology, 2000: 3586-3588*.*
Birkedal-Hansen H, (1993); Role of matrix metalloproteinases in human periodontal diseases. J Periodontol 64(5 Suppl):474-84.
Birkedal-Hansen H, Moore WG, Bodden MK, Windsor LJ, Birkedal-Hansen B, DeCarlo A & Engler JA, (1993); Matrix metalloproteinases: a review. Crit.Rev.Oral Biol.Med. 4, 197-250.
Breimer, D. D., Danhof, M. (1980), Saliva: a fluid for measuring drug concentrations. Pharmacy International. 1, 9-11.
Cimasoni, G. (Ed.). (1974); Monographs in oral science. The crevicular fluid (Vol. 3). Basel: Karger, S.
Cooksley S, Hipkiss JB, Tickle SP, Holmes IE, Docherty AJ, Murphy G & Lawson AD (1990) Immunoassays for the detection of human collagenase, stromelysin, tissue inhibitor of metalloproteinases (TIMP) and enzyme-inhibitor complexes. Matrix 10, 285-291.
Cooper T W, Eisen A Z, Stricklin G P & Welgus H G (1985) Platelet-derived collagenase inhibitor: characterization and subcellular localization. Proc.Natl.Acad.Sci.U.S.A. 82, 2779-2783.
Garrett et al.(1991) Clinical Cancer Research, vol. 7, pages 3450 - 3456.
Goldberg GI, Strongin A, Collier IE, Genrich LT & Marmer BL (1992); Interaction of 92-kDa type IV collagenase with the tissue inhibitor of metalloproteinases prevents dimerization, complex formation with interstitial collagenase, and activation of the proenzyme with stromelysin. J. Biol. chem. 267, 4583-4591.
Hembry RM, Murphy G & Reynolds JJ (1985) Immunolocalization of tissue inhibitor of metalloproteinases (TIMP) in human cells. Characterization and use of a specific antiserum. J. Cell Sci. 73, 105-119.
Holten-Andersen MN, Christensen IJ, Nielsen HJ, Stephens RW, Jensen V, Nielsen OH, Sorensen S, Overgaard J, Lilja H, Harris A, Murphy G, Brunner N, (2002); Total levels of tissue inhibitor of metalloproteinases 1 in plasma yield high diagnostic sensitivity and specificity in patients with colon cancer. Clin Cancer Res;8(1):156-64.
Holten-Andersen MN, Murphy G, Nielsen HJ, Pedersen AN, Christensen IJ, Hoyer-Hansen G, Brunner N, Stephens RW, (1999); Quantitation of TIMP-1 in plasma of healthy blood donors and patients with advanced cancer. Br J Cancer; 80(3-4):495-503.
Jung K., Nowak L., Lein M., Henke W., Schnorr D., Loening S. A. (1996) What kind of specimen should be selected for determining tissue inhibitor of metalloproteinase-1 (TIMP-1) in blood? Clin. Chim. Acta, 254: 97-100, 1996.
JP 08 136548
Khokha R, Waterhouse P, Yagel S, Lala PK, Overall CM, Norton G & Denhardt DT, (1989); Antisense RNA-induced reduction in murine TIMP levels confers oncogenicity on Swiss 3T3 cells. Science 243, 947-50.
Kleiner Jr DE, Tuuttila A, Tryggvason K & Stetler-Stevenson WG, (1993); Stability analysis of latent and active 72-kDa type IV collagenase: the role of tissue inhibitor of metalloproteinases-2 (TIMP-2). Biochemistry 32, 1583-1592.
Liotta LA, Steeg PS & Stetler-Stevenson WG, (1991); Cancer metastasis and angiogenesis: An imbalance of positive and negative regulation. Cell 64, 327-36.
Lovgren, L., Valtonen-Andre, C., Marsal, K., Lilja H. and Lundwall, A. (1999) Measurement of prostate-specific antigen and human glandular kallikrein 2 in different body fluids, Journal of Andrology, 20; 348-355
McCarthy K, Maguire T, McGreal G, McDermott E, O'Higgins N, Duffy MJ. (1999) High levels of tissue inhibitor of metalloproteinase-1 predict poor outcome in patients with breast cancer. Int J Cancer; 84: 44-8.
Murphy G, Houbrechts A, Cockett MI, Williamson RA, O'Shea M & Docherty AJ (1991) The N-terminal domain of tissue inhibitor of metalloproteinases retains metalloproteinase inhibitory activity [published erratum appears in Biochemistry 1991 Oct 22; 30(42):10362]. Biochemistry 30, 8097-8102.
Patent abstracts of Japan vol. 1996, no 09, 30 September 1996
Remacle A, McCarthy K, Noel A, Maguire T, McDermott E, O'Higgins N, et al. (2000) High levels of TIMP-2 correlate with adverse prognosis in breast cancer. Int J Cancer 2000; 89: 118-21.
Stetler-Stevenson WG, Hewitt R & Corcoran M, (1996); Matrix metalloproteinases and tumor invasion: from correlation and causality to the clinic. Semin. Cancer Biol. 7, 147-154.
Turan T, Demir S, Aybek H, Atahan O, Tuncay OL, Aybek Z, (2000) Free and total prostate-specific antigen levels in saliva and the comparison with serum levels in men. Eur Urol.; 38(5):550-4
Vignonola, A. M, Riccobono, L., Mirabella, A., Profita, M., Chanez, P., Bellia, V., Mautino, G., D'Accardi, P., Bousquet, J. and Bonsignore, G. (1998) Sputum Metalloproteinase-9/Tissue Inhibitor of Metalloproteinase-1 Ratio Correlates with Airflow Obstruction in Asthma and Chronic Bronchitis; Am. J. Respir. Crit. Care Med., 158, 1945-1950
Vining, R. F., McGinley, R. A, (1985); Hormones in saliva. Critical Reviews in Clinical Laboratory Sciences. 23, 95-146. Welgus HG, Jeffrey JJ, Eisen AZ, Roswit WT & Stricklin GP (1985); Human skin fibroblast collagenase: interaction with substrate and inhibitor. Coll.Relat.Res. 5, 167-179.
Wolff, K, Hay, A. (1991). Methadone in saliva. Clinical Chemistry. 37, 1297-1298.
Young, J. A., Van Lennep, E. W. (1979); Transport in salivary and salt glands. In G. Giebisch, D. C. Tosteson, H. H. Ussing (Eds.), Membrane transport in biology (pp. 563-674). Berlin: Springer-Verlag.
Zhou, W., Sokoll, L.J., Bruzek, D.J., Zhang, L., Velculescu, V.E, Goldin, S.B, Hruban, R.H., Kern, S.E., Hamilton, S.R., Chan, D.W., Vogelstein B. and Kinzler, K.W. (1998), Identifying markers for pancreatic cancer by gene expression analysis, Cancer Epidemiology Biomarkers & Prevention, Vol 7, 109-112.

## Claims

1. A method for detecting and/or screening colorectal cancer in an individual, said method comprising determining a first parameter represented by the concentration of TIMP-1 in saliva from the individual, wherein the presence of the first parameter above a predetermined discrimination value is an indication that the individual has a high likelihood of having colorectal cancer, with the proviso that an individual with a prior history of cancer is excluded.

2. A method according to claim 1, wherein the first parameter is the total concentration of TIMP-1.

3. A method according to claim 1, wherein the first parameter is the combination of the concentration of total TIMP-1 and the concentration of free TIMP-1.

4. A method according to claim 3, wherein the combination is performed by logistic regression analysis.

5. A method according to any of the preceding claims, wherein the discrimination value is determined by determining the total concentration of TIMP-1 in saliva in both a healthy control population and a population with known cancer, thereby determining the discriminating value which identifies the cancer population with a predetermined specificity or a predetermined sensitivity.

6. A method according to any of the preceding claims, wherein the method further comprises determining at least one second parameter representing the concentration of a marker for cancer different from any form of TIMP-1, in an excreta from an individual.

7. A method according to claim 6, wherein the first and second parameter are combined to result in a combined parameter wherein the presence of a concentration of the combined parameter above a predetermined discrimination value is an indication that the individual has a high likelihood of having a cancer .

8. A method according to claim 7, wherein the discrimination value is determined by determining the combined parameter in the at least one excreta in both a healthy control population and a population with known colorectal cancer, thereby determining the discriminating value which identifies the cancer population with a predetermined specificity or a predetermined sensitivity.

9. A method according to claim 7 or 8, wherein the combination of the first and second parameter is performed by logistic regression analysis.

10. A method according to claim 6-9 wherein the at least one second parameter is the concentration of Carcino Embryonic Antigen (CEA).

11. A method according to any of the preceding claims, wherein the determination of the concentration is performed by means of an immunoassay or an active assay.

12. A method according to any of the preceding claims, wherein the immunoassay is an ELISA.

13. A method according to any of the preceding claims, wherein the active assay is zymography.

## Patentansprüche

1. Verfahren zum Erkennen und/oder Screenen des kolorektalen Karzinoms bei einer Person, wobei das Verfahren die Bestimmung eines ersten Parameters, repräsentiert durch die Konzentration von TIMP-1 im Speichel der Person umfasst, wobei die Gegenwart des ersten Parameters oberhalb eines vorbestimmten Unterscheidungswerts ein Anzeichen dafür ist, dass die Person mit großer Wahrscheinlichkeit ein kolorektales Karzinom hat, mit der Maßgabe, dass eine Person mit einem Karzinom in der Anamnese ausgeschlossen ist.

2. Verfahren nach Anspruch 1, wobei der erste Parameter die Gesamtkonzentration von TIMP-1 ist.

3. Verfahren nach Anspruch 1, wobei der erste Parameter die Kombination der Konzentration von Gesamt-TIMP-1 und der Konzentration von freiem TIMP-1 ist.

4. Verfahren nach Anspruch 3, wobei die Kombination mit einer logistischen Regressionsanalyse durchgeführt wird.

5. Verfahren nach einem der vorhergehenden Ansprüche, wobei der Unterscheidungswert durch Bestimmen der Gesamtkonzentration von TIMP-1 im Speichel in sowohl einer gesunden Kontrollpopulation als auch einer Population mit einem bekannten Karzinom bestimmt wird, sodass der Unterscheidungswert bestimmt wird, mit dem die Karzinompopulation über eine vorbestimmte Spezifizität oder eine vorbestimmte Sensitivität identifiziert wird.

6. Verfahren nach einem der vorhergehenden Ansprüche, wobei das Verfahren weiterhin das Bestimmen mindestens eines zweiten Parameters in einer Ausscheidung einer Person umfasst, der die Konzentration eines Karzinommarkers darstellt, der sich von jeder Form von TIMP-1 unterscheidet.

7. Verfahren nach Anspruch 6, wobei der erste und der zweite Parameter zu einem Kombinationsparameter kombiniert werden, wobei die Gegenwart einer Konzentration des Kombinationsparameters oberhalb eines vorbestimmten Unterscheidungswerts ein Anzeichen dafür ist, dass die Person mit großer Wahrscheinlichkeit ein Karzinom hat.

8. Verfahren nach Anspruch 7, wobei der Unterscheidungswert durch Bestimmen des Kombinationsparameters in der mindestens einen Ausscheidung in sowohl einer gesunden Kontrollpopulation als auch einer Population mit einem bekannten kolorektalen Karzinom bestimmt wird, sodass der Unterscheidungswert bestimmt wird, mit dem die Karzinompopulation über eine vorbestimmte Spezifizität oder eine vorbestimmte Sensitivität identifiziert wird.

9. Verfahren nach Anspruch 7 oder 8, wobei die Kombination des ersten und des zweiten Parameters mit einer logistischen Regressionsanalyse durchgeführt wird.

10. Verfahren nach Anspruch 6-9, wobei der mindestens zweite Parameter eine Konzentration des carcinoembryonalen Antigens (CEA) ist.

11. Verfahren nach einem der vorhergehenden Ansprüche, wobei die Bestimmung der Konzentration mithilfe eines Immunassays oder eines aktiven Assays durchgeführt wird.

12. Verfahren nach einem der vorhergehenden Ansprüche, wobei das Immunassay ein ELISA ist.

13. Verfahren nach einem der vorhergehenden Ansprüche, wobei das aktive Assay eine Zymographie ist.

## Revendications

1. Méthode de détection et/ou de dépistage du cancer colorectal chez un individu, ladite méthode comprenant la détermination d'un premier paramètre représenté par la concentration de TIMP-1 dans la salive de l'individu, dans laquelle la présence du premier paramètre au-dessus d'une valeur discriminante prédéterminée indique que l'individu présente une forte probabilité d'être atteint de cancer colorectal, à condition qu'un individu présentant des antécédents de cancer soit exclu.

2. Méthode selon la revendication 1, dans laquelle le premier paramètre est la concentration totale de TIMP-1.

3. Méthode selon la revendication 1, dans laquelle le premier paramètre est la combinaison de la concentration de TIMP-1 total et de la concentration de TIMP-1 libre.

4. Méthode selon la revendication 3, dans laquelle la combinaison est réalisée par analyse de régression logistique.

5. Méthode selon l'une quelconque des revendications précédentes, dans laquelle la valeur discriminante est déterminée en déterminant la concentration totale de TIMP-1 dans la salive d'une population témoin saine et d'une population que l'on sait atteinte de cancer, en déterminant ainsi la valeur discriminante qui identifie la population cancéreuse avec une spécificité prédéterminée ou une sensibilité prédéterminée.

6. Méthode selon l'une quelconque des revendications précédentes, dans laquelle la méthode comprend également la détermination d'au moins un deuxième paramètre représentant la concentration d'un marqueur de cancer différent de toute forme de TIMP-1 dans un excreta provenant d'un individu.

7. Méthode selon la revendication 6, dans laquelle le premier et le deuxième paramètre sont combinés pour obtenir un paramètre combiné et dans laquelle la présence d'une concentration du paramètre combiné supérieure à une valeur discriminante prédéterminée indique que l'individu présente une forte probabilité d'être atteint de cancer.

8. Méthode selon la revendication 7, dans laquelle la valeur discriminante est déterminée en déterminant le paramètre combiné dans au moins un excreta chez une population témoin saine et une population que l'on sait atteinte de cancer colorectal, en déterminant ainsi la valeur discriminante qui identifie la population cancéreuse avec une spécificité prédéterminée ou une sensibilité prédéterminée.

9. Méthode selon la revendication 7 ou 8, dans laquelle la combinaison du premier et du deuxième paramètre est réalisée par analyse de régression logistique.

10. Méthode selon les revendications 6 à 9, dans laquelle l'au moins un deuxième paramètre est la concentration d'antigène carcino-embryonnaire (ACE).

11. Méthode selon l'une quelconque des revendications précédentes, dans laquelle la détermination de la concentration est réalisée au moyen d'un immunodosage ou d'un dosage d'activité.

12. Méthode selon l'une quelconque des revendications précédentes, dans laquelle l'immunodosage est un test ELISA.

13. Méthode selon l'une quelconque des revendications précédentes, dans laquelle le dosage d'activité est une zymographie.
